(19) Europäisches Patentamt
European Patent Office
Office européen des brevets

(11) **EP 0 753 741 A1**

(12) **EUROPEAN PATENT APPLICATION**
published in accordance with Art. 158(3) EPC

(43) Date of publication:
15.01.1997 Bulletin 1997/03

(51) Int. Cl.$^6$: **G01N 33/48**

(21) Application number: 96901133.7

(86) International application number:
PCT/JP96/00159

(22) Date of filing: 29.01.1996

(87) International publication number:
WO 96/24058 (08.08.1996 Gazette 1996/36)

(84) Designated Contracting States:
DE GB

(30) Priority: 30.01.1995 JP 13221/95
16.05.1995 JP 117488/95

(71) Applicant: Niigata Engineering Co., Ltd.
Ohta-ku, Tokyo 144 (JP)

(72) Inventors:
• TSUKAGOSHI, Youichi
Tokyo 176 (JP)
• YAMAMOTO, Toshimasa
Kawasaki-shi Kanagawa 214 (JP)

(74) Representative: Woodward, John Calvin et al
Venner Shipley & Co.
20 Little Britain
London EC1A 7DH (GB)

(54) **COMPONENT SEPARATION MEMBER AND COMPONENT SEPARATOR EQUIPPED WITH SAID MEMBER**

(57) This component separating member comprises a member main body (44) inserted into a tubular container (42), and at least one ring-shaped elastic member (45), impermeable to liquids, which is provided at an outer circumferential portion of an upper portion of the member main body (44) and which presses against an inner wall of the container (42).

With this component separating member, a reliable seal is able to be obtained even at arbitrary positions on blood collecting tubes having tapered tube diameters, such as plastic blood collecting tubes, and even if the container is left to sit for a long period of time after separation, it can prevent one component from shifting into the other component so as to maintain these components in a state of complete separation.

FIG.1

## Description

TECHNICAL FIELD

The present invention relates to component separating member and component separating tube provided therewith, which are suitable for use in separating the plurality of components in a liquid sample by means of a centrifugal separation operation, especially in separating serum and clot in blood specimens by means of a centrifugal separation operation.

BACKGROUND ART

Conventionally, separating member is used to stabilize the state of separation when a liquid sample is separated by means of a centrifugal separation operation.

As method for setting this separating member onto test tubes or the like, there is a post-insertion method wherein the separating member is inserted after injecting liquid sample into the test tube, and a pre-insertion method wherein the separating member is pre-inserted into the test tube; in both cases, the separating members must be affixed within the test tubes before the centrifugal separation.

The reason for this is that, in the post-insertion method for example, a centrifugal separation operation is sometimes started without affixing the separating member to the test tube, in which case the separating member sink inside the test tube immediately after the separating member is inserted within the test tube or even due to the slight centrifugal force when the centrifugal separation operation has been started, by which they slide along the inner wall of the test tube so as to rub the part of the liquid sample attached to the inner wall which is difficult to peel or drop by means of a slight centrifugal force, thereby resulting in inaccurate analysis values.

Here, a blood separation operation will be explained as an example of a centrifugal separation operation.

Since blood tests are mainly performed by using the serum (blood plasma) separated from the whole blood, pre-processes of the test must be conducted in order to separate the blood specimens, collected into blood collecting tubes such as spitzes, into blood serum and blood plasma (hereinafter referred to simply as serum), and blood clot or blood cells (hereinafter referred to simply as clot).

Usually, this blood separation operation is performed by putting collected blood sample into blood collecting tube such as spitz, then separating these by centrifugal separation into serum as test specimens. However, with this blood separation operation, the state of separation between the serum and the clot is extremely unstable, so that even a slight bump can mix blood cells from the precipitated clot into the serum, thus requiring considerably careful operation when handling the blood specimen after separation.

Numerous methods have been tested to improve the unstable state of separation of the blood specimens after centrifugal separation in order to simplify the testing procedures, and one example is to use a thixotropic serum separating agent having a specific gravity between those of the serum and the clot.

This serum separating agent may, for example, be composed primarily of a synthetic resin of low molecular weight such as silicone oil, having a constant specific gravity and thixotropy, which flows during centrifugal separation to form a firm partition above the clot.

However, this type of serum separating agent has the following drawbacks.

(1) When the physical properties of the clot is not normal, a strong partition cannot be formed. For example, when the specific gravity is low and the clot is soft as is often seen in dialysis patients, the serum and the clot cannot completely separate.

(2) Since the serum separating agents are hydrophobic, they are insoluble in water and dissolvable with oil-soluble chemicals. Therefore, when the chemical concentration in the blood is measured, the chemicals often dissolve into the serum separating agent and result in incorrect analysis values.

(3) When testing for blood coagulation factors, a serum separating agent cannot be used because the serum separating agent can activate the blood coagulation factors.

(4) The properties of blood differ depending upon the individual. Specifically, there is a need to contain fibrin because the generation of fibrin hinders the analysis of the serum after centrifugal separation, but fibrin cannot be contained by the serum separating agent. Since fibrin is especially likely to be generated in the blood of dialysis patients, the generation of fibrin cannot be prevented by the serum separating agents.

Therefore, in order to resolve these problems of the serum separating agents, a serum filtering piston such as shown in Fig. 26 has been proposed (see Japanese Patent Application, First Publication No. 51-105890).

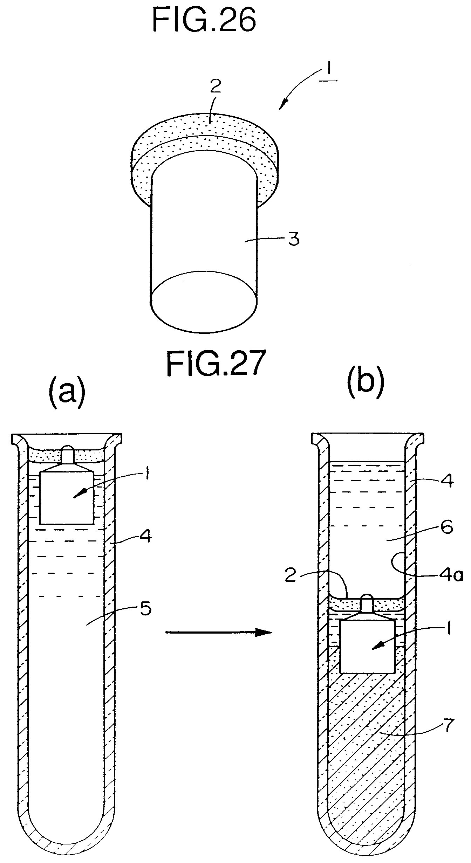

This serum filtering piston 1 is composed of a disc-shaped filter 2 having a diameter slightly larger than the inner diameter of the blood collecting tube, and a cylindrical weight 3 attached to the center of the filter 2 having a diameter less than that of the filter 2. As shown in Fig. 27(a), after putting the collected blood 5 into the blood collecting tube 4, the serum filtering piston 1 is inserted into the blood collecting tube 4. Then, by performing a centrifugal separation, this serum filtering piston 1 moves to the boundary between the-serum 6 and the clot 7 as shown in Fig. 27(b), and the filter 2 adheres to the inner walls 4a of the blood collecting tube 4 to completely separate the serum 6 from the clot 7 so as to allow the serum to be withdrawn by decantation.

On the other hand, while vacuum blood collecting tubes wherein rubber stoppers are fitted into the mouths of conventional blood collecting tubes in a vacuum are used as separating containers for separating serum, in recent years, vacuum blood collecting tubes have been proposed wherein a gas barrier film such as an aluminium film is fused to the mouths of the blood collecting tubes in vacuum and thin rubber leaves are adhered to the center of the film in order to improve the tightness of the seal.

However, the above-mentioned serum filtering piston 1 has a structure wherein the filter 2 is pinned to the weight 3, so that the filter 2 may become detached. Additionally, since the filter 2 is pulled down by the piston 1, the filter 2 must have a certain level of strength so as to allow it to move downward while bending to slide along the inner wall 4a of the blood collecting tube 4. For this reason, blood cells may be damaged by rubbing the clot stuck to the inner wall 4a, thus resulting in errors in test values such as the LDH (lactate dehydrogenase) count.

Additionally, the filter 2 tends to move jerkily, as a result of which there are problems in that it is difficult to balance the filter 2 horizontally, so that blood corpuscles in the clot 7 can pass through the gap between the inner wall 4a and the filter 2 to mix into the serum 6 when the serum is collected after centrifugal separation, or the clot can become stuck above the filter 2.

Furthermore, this filter 2 is water-permeable and therefore cannot completely separate the components of the serum 6 and the clot 7; for example, the clot components such as potassium (K) can shift into the serum after separation.

The above-mentioned vacuum blood collecting tube with a rubber stopper has drawbacks in that the area of contact between the blood collecting tube and the rubber stopper must be large in order to maintain airtightness and the rubber stopper must be pressed by a strong force, thus making the rubber stopper itself bulky or making it difficult to remove the rubber stopper. Additionally, the so-called pop-up phenomenon cannot be prevented with rubber stoppers. The pop-up phenomenon occurs when a stopper which has once been removed is re-engaged; the air inside the container is compressed when the stopper is engaged, and the stopper eventually comes loose due to expansion of the compressed air.

Additionally, with vacuum blood collecting tubes with gas-barrier films, extra work is required to manually remove the film when the serum is poured into a different container after centrifugal separation.

Therefore, the present inventors and others have proposed the following type of blood separating member.

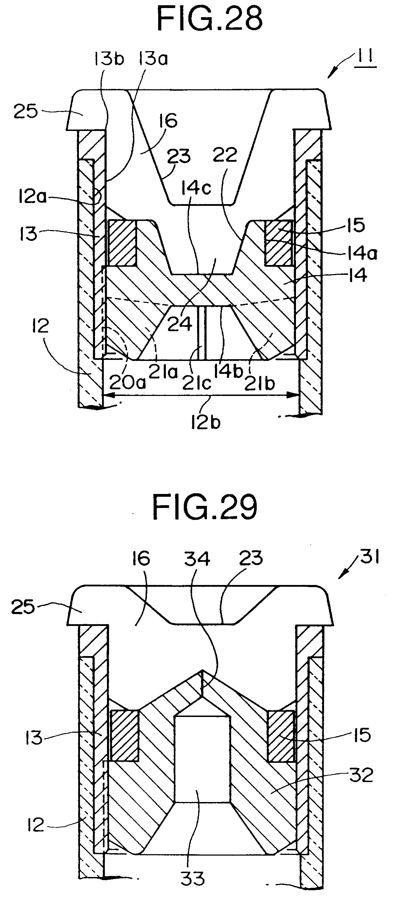

Fig. 28 is a vertical section view of a blood separating member 11; in the drawing, reference numeral 12 denotes a blood collecting tube (tubular container) formed from glass or a plastic such as polyethylene terephthalate (PET) to which a blood separating member 11 is applied, reference numeral 13 denotes a tubular member fitted into the mouth portion 12a of the blood collecting tube 12, reference numeral 14 denotes a member main body inserted into the tubular member 13, reference numeral 15 denotes a ring-shaped partition member fitted into the upper circumferential portion 14a of the member main body 14, and reference numeral 16 denotes a cap which fits with the member main body 14 and the tubular member 13.

The blood separating member 11 is composed of the tubular member 13, the member main body 14, the partition member 15 and the cap 16.

The tubular member 13 has an inner diameter slightly less, for example 0.2 ~ 3 mm less, than the inner diameter of the mouth portion 12a of the blood collecting tube 12, and the inner wall has a plurality of grooves 20a ~ 20c symmetric with respect to the central axis formed at three locations in the axial direction for allowing passage of air. A hard plastic such as polycarbonate or polyethylene terephthalate resin can be used as the material for this tubular member 13.

The member main body 14 is an elastic disc having a central portion which thins in the vertical direction so as to allow a blood collection needle to pass easily, wherein the bottom surface 14b side has a plurality of grooves 21a ~ 21c for passage of air formed from the central axis to the circumferential surface at three locations symmetric with respect to the central axis, and wherein a fitting concave portion 22 having a cross-sectional trapezoidal shape for fitting with the cap 16 is formed along the central axis at the central portion of the upper surface 14c.

The partition member 15 is formed either from an elastic and liquid-permeable material or a material which swells due to the serum, and has a cross-sectional rectangular shape with a diameter slightly less than the above-mentioned member main body 14, wherein the diameter of the hole in the central portion is somewhat smaller than the outer diameter of the circumferential portion 14a of the member main body 14.

The cap 16 is molded into an approximate cylindrical shape from butyl rubber or the like having elasticity and excellent gas-barrier properties; the top central portion is formed into a concave portion 23 so as to allow a blood collection needle to easily pass through, a fitting convex portion 24 having a cross-sectional trapezoidal shape for fitting with the fitting concave portion 22 is formed at a bottom portion, and a ring-shaped fitting portion 25 for fitting with the mouth portion 13b of the tubular member 13 is formed at a peripheral portion of the upper portion.

With this blood separating member 11, the serum 6 and the clot 7 can be completely separated, and the blood cells in the clot 7 will not mix into the serum 6. Furthermore, the serum 6 and the clot 7 can be completely separated even if there are changes in the inner diameter of the blood collecting tube 12.

Additionally, blood separating members 31 such as shown in Fig. 29 have been proposed.

In Fig. 29, the constituent elements identical to those of the blood separating member 11 of Fig. 28 are given the same reference numbers and their explanation is omitted.

A coaxial space portion 33 for holding air having an open bottom is formed in the central portion of the member main body 32 of the blood separating member 31, and a cut 34 which allows passage in the vertical direction is formed above the space portion 33 in the central portion of the member main body 32.

This cut 34 is not an aperture such as a through hole, and may be cut by a sharp blade such as a carving blade. It is normally closed, but may open slightly when a certain load is applied such as during centrifugal separation.

This blood separating member 31 allows smooth movement between the member main body 32 and the clot.

However, while the above-mentioned blood separating member 11 is effective in completely separating the serum 6 and the clot 7 by performing centrifugal separation after collecting blood, if the blood collecting tube 12 is left in a refrigerator for approximately one week after separation, components such as potassium (K) in the clot 7 can penetrate the blood separating member 11 and mix into the serum.

This is due to the fact that the partition member 15 is formed either from a elastic and liquid-permeable material or a material which swells with the serum, so that if left for a long period of time within the blood collecting tube 12 after separation, the permeable components in the clot 7 dissolve away from the clot 7 and penetrate the partition member 15.

Additionally, the above-mentioned blood separating member 31 sinks to the boundary between the serum and the clot while releasing the air from the space portion 33 during centrifugation, then is sealed with the air removed to completely separate the serum and the clot, so as to prevent shifting of the clot components to the serum, but the speed at which the separating member 31 sinks in the serum during centrifugation cannot be controlled, so that there is a problem in that the shock on the clot can be large.

Controlling the sinking speed of the separating member 31 within the serum during centrifugation is an extremely crucial point in order to effectively separate the serum.

Additionally, with the above-described blood separating member 11, it is difficult to obtain a reliable sealing effect at an arbitrary position in the blood collecting tube, especially with blood collecting tubes which have tapered tube diameters such as plastic blood collecting tubes.

The present invention has been made in consideration of the above-mentioned situations, offers a component separating member and a component separating tube provided therewith, which can completely ensure airtightness above and below the member when the member is applied to a container in the handling of liquid samples, especially the handling of blood specimens, wherein there is no risk of the separating member adhering to the wall surface of the container such as a blood collecting tube even when left for a long period of time, so as to result in more accurate analysis figures.

DISCLOSURE OF THE INVENTION

The component separating member according to the present invention comprises a member main body inserted into a tubular container; and at least one ring-shaped elastic member, provided at an outer circumferential portion of an upper portion of the member main body, which presses against an inner wall of the container and is impermeable to liquids.

When this component separating member is fitted into the container and a centrifugal separation is performed after putting a collected liquid sample inside the container, the elastic member slides along the inner wall of the container so as to control the sinking speed of the member main body in the centrifugally separated components. Therefore, the member main body drops slowly inside the container, moves to the boundary between the plurality of centrifugally separated components, and presses against one of the components without causing any disturbances. As a result, the member main body is stopped at a designated portion, and one component is prevented from moving into the other component. Additionally, since the elastic member is impermeable to liquids, even if the container is left to sit for a long period of time after separation, one component is prevented from shifting into the other component so as to maintain these components in a state of complete separation.

With this component separating member, even if the container is left to sit for a long period of time after separation, one component can be prevented from shifting into the other component so as to maintain these components in a state of complete separation.

Another component separating member according to the present invention has at least one elastic portion, formed at an outer circumferential portion of an upper portion of a member main body inserted into a tubular container, which completes a circuit in the circumferential direction and presses against the inner wall of the container.

When a centrifugal separation is performed on a collected liquid sample using this component separating member, the elastic member presses against the inner wall of the container due to the elastic force, the member main body is stopped at a designated position, and one of the components is prevented from moving to the other component layer, as with the above-mentioned component separating member. Consequently, the member main body will not shift from the designated position during centrifugal separation, so that these components are completely separated.

With this component separating member, one of the components can be prevented from moving to the other component layer due to the member main body stopping at a designated position. Consequently, the member main body will not shift from the designated position during centrifugal separation, so that these components can be completely separated.

Another component separating member according to the present invention comprises a member main body inserted into a tubular container; and a ring-shaped elastic member which is supported on an outer circumferential portion of the member main body so as to be capable of moving in an axial direction of the member main body, and which presses against an inner wall of said container; wherein a first groove for supporting the elastic member in a state of protrusion outward from the outer circumferential portion and a second groove at a position above the first groove for housing the elastic member are respectively formed on an outer circumferential portion of the member main body.

Whether the format of this component separating member is a post-insertion format or a pre-insertion format, the state prior to performing a centrifugal separation, that is, the state wherein the collected liquid sample is sealed within the container along with the component separating member is the same. In this case, the inner circumferential side of the elastic member is fitted with the first groove of the member main body, and the outer circumferential side protrudes further outward than the outer circumferential portion of the member main body so as to press against the inner wall of the container.

Subsequently, centrifugal separation is performed. When the centrifugal separation operation starts and the centrifugal force exceeds a designated value, the centrifugal force causes the member main body to drop and to shift slightly downward while the elastic member is pressed against the inner wall of the container, thereby releasing the member main body from the first groove. As the member main body gradually drops, the second groove moves to the position of the elastic member so as to house the elastic member. This housed elastic member no longer presses against the inner wall of the container, as a result of which the resistance force no longer opposes the sinking of the member main body so that it quickly moves to the boundary between the plurality of centrifugally separated components and presses the components downward for separation.

Due to the above, the areas above and below the member can be held completely airtight when the member is attached to the container prior to centrifugal separation, and the component separating member will not adhere to the wall surface of the container when left for a long period of time. Consequently, more accurate analysis values can be determined.

Specifically, the following five points can be given as features of the present invention.

(1) Since the structure is such as to allow centrifugal separation with air held in the space portion, the air acts as a cushion to weaken the impact of the blood separating member on the blood clot, and the sinking speed can be controlled by changing the apparent specific gravity of the member due to compression of the air during centrifugation.
(2) The top surface of the elastic portion of the member is curved and the neck diameter is reduced so as to smoothen the motion of the elastic portion, thereby allowing a reliable seal to be obtained at an arbitrary position on the tapered blood collecting tube.
(3) Since the material of the elastic portion has the properties of both elasticity and plasticity, the sealing property inside the blood collecting tube can be made stronger and more reliable.
(4) An elliptical O-ring allows the member to be easily and reliably affixed to a top end of the blood collecting tube.
(5) The impact on the clot can be reduced and the serum recovery rate can be increased by providing inert particles below the member.

BRIEF EXPLANATION OF THE DRAWINGS

Fig. 1 is a vertical section view showing a component separating member according to a first embodiment of the present invention.
Fig. 2 is a step diagram showing a centrifugal separation operation of a liquid sample with a component separating member according to a first embodiment of the present invention.
Fig. 3 is a vertical section view showing a modification example of a component separating member according to a first embodiment of the present invention.
Fig. 4 is a vertical section view showing a component separating member according to a second embodiment of the present invention.
Fig. 5 is a single step diagram showing a centrifugal separation operation of a liquid sample with a component separating member according to a second embodiment of the present invention.
Fig. 6 is a vertical section view showing a modification example of a component separating member according to a second embodiment of the present invention.

Fig. 7 is a vertical section view showing a component separating member according to a third embodiment of the present invention.

Fig. 8 is a single step diagram showing a centrifugal separation operation of a liquid sample with a component separating member according to a third embodiment of the present invention.

Fig. 9 is a vertical section view showing a component separating member according to a fourth embodiment of the present invention.

Fig. 10 is a vertical section view showing a blood collecting tube.

Fig. 11 is a vertical section view showing a first modification example of a component separating member according to a fourth embodiment of the present invention.

Fig. 12 is a vertical section view showing a second modification example of a component separating member according to a fourth embodiment of the present invention.

Fig. 13 is a vertical section view showing a third modification example of a component separating member according to a fourth embodiment of the present invention.

Fig. 14 is a top view showing a third modification example of a component separating member according to a fourth embodiment of the present invention.

Fig. 15 is a vertical section view showing a fourth modification example of a component separating member according to a fourth embodiment of the present invention.

Fig. 16 is a vertical section view showing a component separating member according to a fifth embodiment of the present invention.

Fig. 17 is a vertical section view showing a component separating member according to a sixth embodiment of the present invention.

Fig. 18 is a vertical section view showing a component separating member according to a seventh embodiment of the present invention.

Fig. 19 is a vertical section view showing a component separating tube attached with a component separating member according to an eighth embodiment of the present invention.

Fig. 20 is a step diagram showing a centrifugal separation operation of a liquid sample with a component separating member according to an eighth embodiment of the present invention.

Fig. 21 is a vertical section view showing a component separating tube attached with a component separating member according to a modification example of an eighth embodiment of the present invention.

Fig. 22 is a vertical section view showing a component separating tube attached with a component separating member according to a ninth embodiment of the present invention.

Fig. 23 is a vertical section view showing a component separating member according to a tenth embodiment of the present invention.

Fig. 24 is a perspective view showing an elliptical O-ring of a component separating member according to a tenth embodiment of the present invention.

Fig. 25 is a single step diagram showing a centrifugal separation operation of a liquid sample with a component separating member according to a tenth embodiment of the present invention.

Fig. 26 is a perspective view showing a conventional serum filtering piston.

Fig. 27 is a step diagram showing a centrifugal separation operation of a liquid sample due to a conventional serum filtering piston.

Fig. 28 is a vertical section view showing an example of a conventional improved component separating member.

Fig. 29 is a vertical section view showing another example of a conventional improved component separating member.

BEST MODE FOR CARRYING OUT THE INVENTION

Hereinbelow, the embodiments of the component separating member and component separating tube provided therewith according to the present invention will be explained.

[First Embodiment]

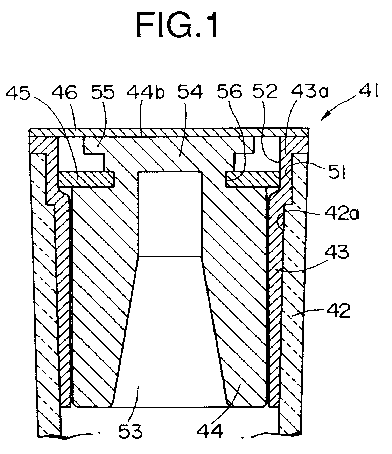

Fig. 1 is a vertical section view showing a blood separating member (component separating member) 41 according to a first embodiment of the present invention. In the drawing, reference numeral 42 denotes a blood collecting tube (tubular container), reference numeral 43 denotes a tubular member fitted into the mouth portion 42a of the blood collecting tube 42, reference numeral 44 denotes a member main body inserted into the tubular member 43, reference numeral 45 denotes a ring-shaped elastic member fitted into the top circumferential portion of the member main body 44, reference numeral 46 denotes a thin sheet formed from natural rubber or the like which covers the top surface 44b of the member main body 44 and is sealed onto the top end portion 43a of the tubular member 43 by adhesives or the like.

The blood separating member 41 comprises a tubular member 43, a member main body 44 and an elastic member 45.

The blood collecting tube 42 can be composed of glass or a plastic such as polyethylene terephthalate (PET), and a concave portion 51 is formed around the entire circumference of the mouth portion 42a.

The tubular member 43 has an inner diameter which is slightly less, for example 0.2 ~ 3 mm less, than the inner diameter of the mouth portion 42a of the blood collecting tube 42; the top end portion 43a has an expanded diameter so as to fit with the concave portion 51, and has an inner circumferential surface which has a concave portion 52 formed around the entire circumference.

The material of this tubular member 43 can be a hard plastic with good gas-barrier properties, such as polycarbonate or polyethylene terephthalate resin.

In this case, the difference between the inner diameters of the blood collecting tube 42 and the tubular member 43 is 0.2 ~ 3 mm, but if the blood collecting tube 42 is plastic, the inner diameter towards the bottom is less than that towards the top due to tapering, so that the inner diameter of the blood collecting tube should be considered for a minimum amount of blood.

The member main body 44 is roughly tubular, having a space portion 53 which opens downward formed coaxially at a central portion thereof, and the roof plate 54 above this space portion 53 is thinned in the vertical direction so as to allow a blood collecting needle to pass easily therethrough. The top end portion of this member main body 44 widens outward in a horizontal direction to form an annular portion 55, and a groove 56 is formed around the entire circumference slightly thereunder to fit an elastic member 45.

The member main body 44 has a specific gravity which is between those of the serum and the clot, is easily punctured by a blood collecting needle, and is soft and elastic so as to be capable of closing up after a blood collecting needle has been withdrawn. Specifically, rubbers or elastomers, for example styrene/butadiene rubber, butyl rubber, silicone rubber, polystyrene type elastomers, polyamide type elastomers, or silicone type elastomers, or those wherein inorganic materials such as barium sulfate have been mixed into these materials to adjust the specific gravity can be used. The hardnesses of the rubber and elastomers should be approximately 30 ~ 60 degrees according to the JISA (JISK6301).

The elastic member 45 is composed of an elastic non-liquid-permeable material which does not allow passage of blood serum, formed into a ring having a rectangular cross section with a diameter greater than the diameter of the member main body 44, which is pressed by a designated elastic force at the concave portion 52 of the tubular portion 43, and has a hole in the central portion with a diameter slightly less than the diameter of the groove 56 portion of the member main body 44.

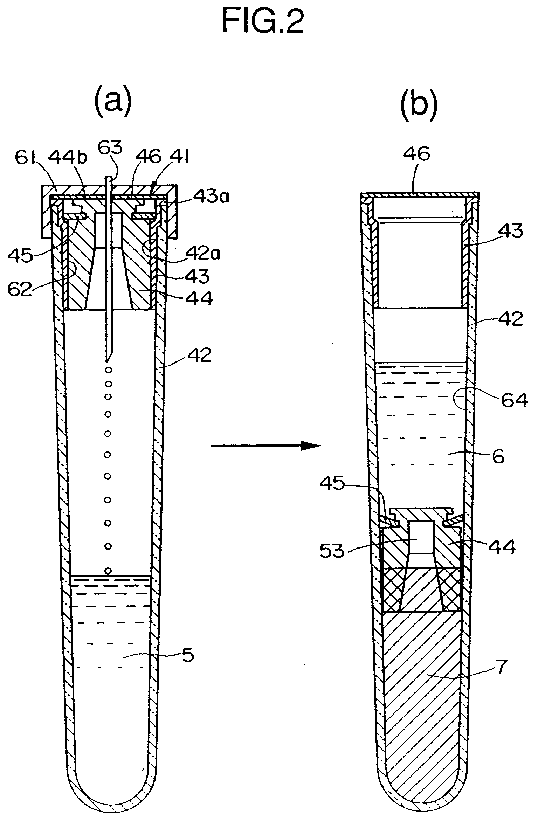

Next, a method for separating blood using this blood separating member 41 will be explained with reference to Fig. 2.

First, a blood separating member 41 is attached to the mouth portion 42a of the blood collecting tube 42, then a sheet 46 which covers the top surface 44b of the member main body 44 is sealed onto the top end portion 43a of the tubular member 43 by means of adhesive or the like, and the cap 61 is attached (Fig. 2A). In this case, the member main body 44 must be pressed against the inner wall 62 of the tubular member 43 at a designated pressure or greater, so as not to easily slide from the tubular member 43. The interior of this blood collecting tube 42 is held in a reduced pressure state so as to be a vacuum blood collecting tube.

Next, this blood separating member 41 is punctured from above by a blood collecting needle 63, and the collected blood 5 is injected. The amount of blood injected is determined by the degree of depressurization inside the blood collecting tube 42, but is always positioned beneath the member main body 44, and does not go above it.

Subsequently, this blood collecting tube 42 is left to rest indoors, so as to coagulate the blood.

Thereafter, centrifugal separation is performed (Fig. 2B). The member main body 44 slides down the inner wall 62 of the tubular member 43 due to the centrifugal force, but the force of buoyancy on the member main body 44 due to the blood 5 is zero until the member main body 44 separates from the tubular member 43. Therefore, if the magnitude of the applied centrifugal force is 1000 g for example, then the member main body 44 receives the same force as if sinking through the air at 1000 g, and easily separates from the tubular member 43. During this time, air fills the space portion 53.

As the centrifugal separation progresses further so that the member main body 44 separates from the tubular member 43, the sinking speed of the member main body 44 in the serum 6 is controlled by the elastic member 45 sliding along the inner wall 64 of the blood collecting tube 42. Consequently, the member main body 44 slowly sinks inside the blood collecting tube 42, moves to the boundary between the serum 6 and the clot 7, and presses onto the clot 7 without causing any disturbances. As a result, the member main body 44 is affixed to a designated position inside the blood collecting tube 42, i.e. the boundary between the serum 6 and the clot 7, thereby preventing the clot 7 from shifting into the serum 6 layer. Additionally, since the elastic member 45 is non-liquid-permeable, the components in the clot 7 are obstructed from shifting into the serum 6 even when the blood collecting tube 42 is left to stand for a long period of time after separation, thus holding the serum 6 and the clot 7 in a completely separated state.

This blood separating member 41 is able to be moved to the boundary between the serum and the clot by centrifugal force with air held in the space portion 53, by choosing appropriate shapes for the space portion 53 and specific gravities of the member main body 44, thereby containing the clot. In this case, the air in the space portion 53 acts as a cushion to weaken the collision force on the clot, so as to prevent destruction of blood corpuscles. Furthermore, the roof plate 54 of the space portion 53 does not allow air to escape, so that components can be completely prevented from shifting into the serum.

The air inside the space portion 53 is compressed depending upon the centrifugal force (such as 1000 g or 1500 g), the amount of blood (such as 3 ml or 9 ml) and the position of the member (the distance from the center of the centrifuge to the member). While the average specific gravity of the entire member including the compressed air differs according to the centrifugal force, amount of blood and position of the member, separation of the serum and the clot is possible as long as it is between the serum and the clot in the range of operating conditions normally applied to the serum separation.

In this case, the sinking speed of the member inside the serum can be controlled by changing the average specific gravity of the entire member including compressed air during centrifugation so as to achieve a good state of separation between the serum and clot.

For example, the member main body 44 can have an outer diameter of 12.2 mm and a height of 16 mm, the width of the grooves at the portion for fitting the elastic member 45 can be 2.5 mm, the space portion can have an inner diameter of 4 mm and a depth of 14 mm, and the specific gravity of the entire member can be 1.2.

The pressure of the air inside the space portion 53 returns to approximately atmospheric pressure when the centrifugation is completed, and expands to return to the original volume, but the elastic member 45 is stuck to the inner wall of the blood collecting tube 42 at this time, so that the expansion of the air is impeded. As a result, the serum 6 and the clot 7 are able to be completely separated.

Additionally, although blood can be collected by passing through the roof plate 54, air will not leak through the hole left by the passage of the blood collecting needle during centrifugation, so that the member will become positioned at the boundary between the serum and the clot by any type of centrifugation in normal serum separation.

As explained above, this blood separating member 41 comprises a tubular member 43 fitted into a mouth portion 42 of a blood collecting tube 42, a member main body 44 inserted into the tubular member 43, and a ring-shaped elastic member 45 fitted into the top circumferential portion of the member main body 44, so that the sinking speed of the member main body 44 in the blood serum 6 can be controlled by sliding the elastic member 45 along the inner wall 64 of the blood collecting tube 42. Consequently, the member main body 44 can be stopped at a designated position, i.e. the boundary between the clot 7 and the serum 6, thereby preventing the clot 7 from shifting into the serum layer 6.

Additionally, the elastic member 45 is non-liquid-permeable, so that even if the blood collecting tube 42 is left for a long period of time after separation, components in the clot 7 can be obstructed from moving into the serum 6 in order to hold the serum 6 and the clot 7 in a completely separated state.

Furthermore, even if the inner diameter of the blood collecting tube 42 changes, the elastic force of the elastic member 45 presses it against the inner wall 64 so that the serum 6 and the clot 7 are completely separated.

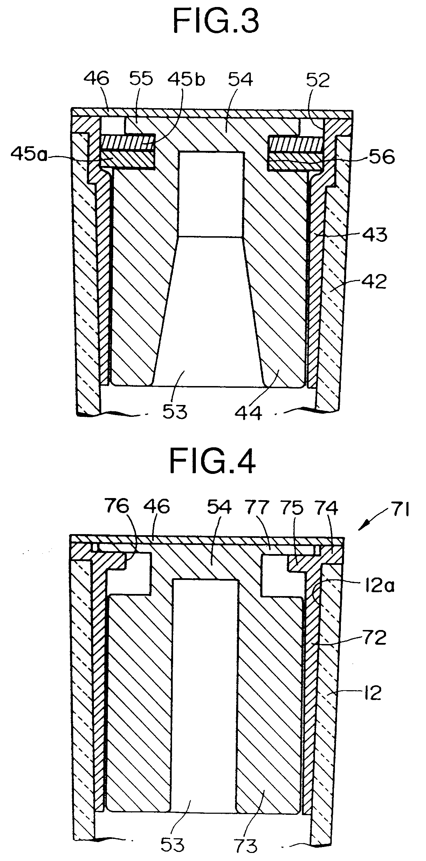

While the blood separating member 41 of the present embodiment has a structure wherein a single elastic member 45 is fitted into the top circumferential portion of the member main body 44, it is possible to have a structure wherein two elastic members 45a and 45b are fitted into a groove 56 widened to the annular portion 55 as shown in Fig. 3.

In this case, two elastic members 45a and 45b slide along the inner wall 64 of the blood collecting tube 42, so that the sinking speed of the member main body 44 in the serum 6 can be further controlled, thereby stopping the member main body 44 at the boundary between the clot 7 and the serum 6 to reliably prevent clot 7 from moving into the serum 6 layer.

[Second Embodiment]

Fig. 4 is a vertical section view showing a blood separating member (component separating member) 71 according to a second embodiment of the present invention. In the drawing, reference numeral 72 denotes a tubular member fitted into a mouth portion 12a of a blood collecting tube 12, and reference numeral 73 denotes a member main body inserted into the tubular member 72.

The blood separating member 71 comprises a tubular member 72 and a member main body 73.

The tubular member 72 has an inner diameter which is slightly less, for example 0.2 ~ 3 mm less, than the inner diameter of the mouth portion 12a of the blood collecting tube 12; the outer circumferential portion 74 of the top end has an expanded diameter so as to fit with the mouth portion 12a of the blood collecting tube 12, has an inner circumferential portion 75 which protrudes horizontally inward, and a large diameter hole 76 formed in the central portion.

The member main body 73 is roughly tubular, and the top circumferential portion of this member main body 73 is a thin leaf 77 which extends horizontally outward.

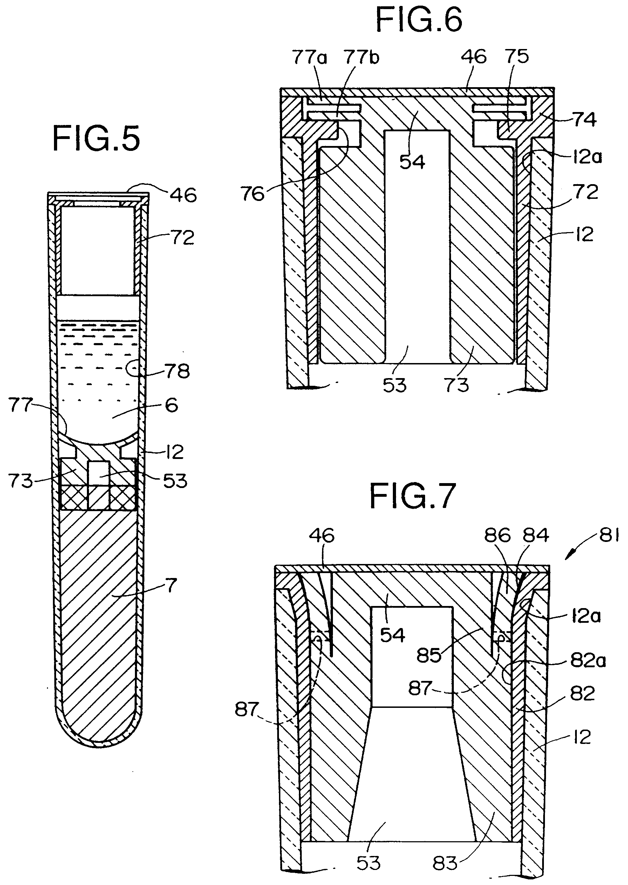

Next, a method for separating blood components using this blood separating member 71 will be explained with reference to Fig. 5.

After the blood separating member 71 is applied to the mouth portion 12a of the blood collecting tube 12, the method is absolutely identical to that of the first embodiment explained above until the blood is coagulated by storing the blood collecting tube 12 indoors, so the explanation will be omitted.

Then, a centrifugal separation is performed. As the member main body 73 drops by sliding along the inner wall 78 of the tubular member 12, it receives a force identical to sinking in air at 1000 g, so as to easily separate from the tubular member 72. During this time, the space portion 53 is filled with air.

As the centrifugal separation progresses further and the member main body 73 separates from the tubular member 72, the thin leaf 77 slides while pressing onto the inner wall 78 of the blood collecting tube 12 so as to control the sinking speed of the member main body 73 in the serum 6, and the member main body 73 contains the clot 7 without causing any disturbances. As a result, the member main body 73 is stopped at the boundary between the serum 6 and the clot 7 to prevent clot 7 from moving into the serum 6.

In exactly the same manner as the blood separating member 41 of the first embodiment, this blood separating member 71 is also capable of controlling the sinking speed of the member main body 73 in the serum 6; consequently, the member main body 73 can be stopped at the boundary between the clot 7 and the serum 6 so as to prevent clot 7 from moving into the serum 6 layer.

While the blood separating member 71 has a single thin leaf 77 extending horizontally outward from the top circumferential portion of the member main body 73, two mutually horizontal thin leaves 77a and 77b can be extended horizontally outward from two areas at the top circumferential portion of the member main body 73 as shown in Fig. 6.

In this case, two thin leaves 77a and 77b slide along the inner wall 78 of the blood collecting tube 12 so as to further control the sinking speed of the member main body 73 in the serum 6, thereby allowing the member main body 73 to be stopped at the boundary between the clot 7 and the serum 6 to reliably prevent clot 7 from moving into the serum 6 layer.

[Third Embodiment]

Fig. 7 is a vertical section view showing a blood separating member (component separating member) 81 according to a third embodiment of the present invention. In the drawing, reference numeral 82 denotes a tubular member fitted into the mouth portion 12a of the blood collecting tube 12, and reference numeral 83 denotes a member main body inserted into the tubular member 82.

The blood separating member 81 comprises a tubular member 82 and a member main body 83.

The tubular member 82 has an inner diameter slightly less, for example 0.2 ~ 3 mm less, than the inner diameter of the mouth portion 12a of the blood collecting tube 12, and the inner circumferential surface 84 of the top end is formed so that the diameter gradually expands horizontally outward in going upward, in a flare shape for example.

The member main body 83 is approximately tubular and has elasticity, with a thin leaf 86 which presses the inner wall 82a of the tubular member 82 formed unitarily around the entire circumference.

This thin leaf 86 extends upward and outward from the outer circumferential portion 85, the inner circumferential surface has an approximate ring shape which separates from the outer circumferential portion 85 in going upward, and a plurality of through holes 87 are formed at uniform intervals around the axis of the thin leaf 86 at the bottom portion near the outer circumferential portion 85.

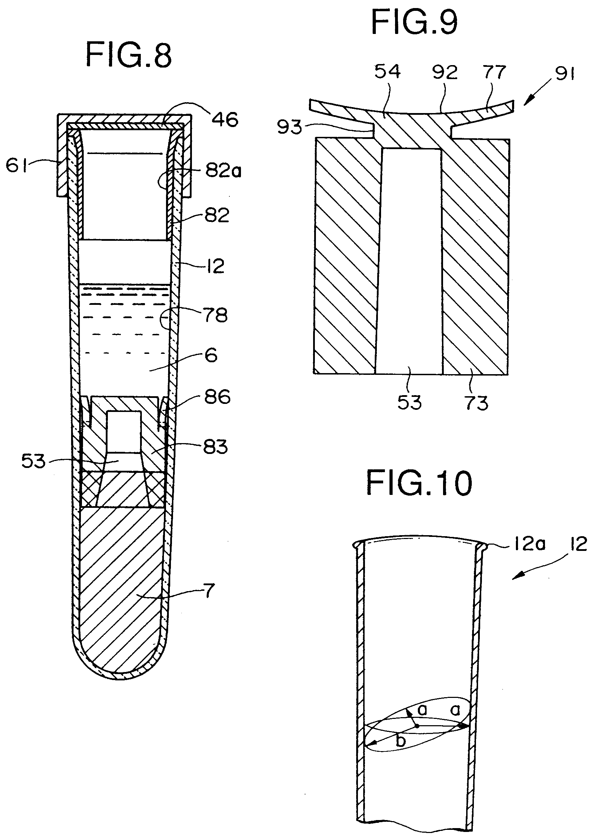

Next, the method for separating blood components using this blood separating member 81 will be explained with reference to Fig. 8.

After the blood separating member 81 is applied to the mouth portion 12a of the blood collecting tube 12, the method is absolutely identical to those of the first and second embodiments explained above until the blood is coagulated by storing the blood collecting tube 12 indoors, so the explanation will be omitted.

Then a centrifugal separation is performed. As the member main body 83 drops by sliding along the inner wall 82a of the tubular member 82, it receives a force identical to sinking in air at 1000 g, so as to easily separate from the tubular member 82. During this time, the space portion 53 is filled with air.

As the centrifugal separation progresses further and the member main body 83 separates from the tubular member 82, the thin leaf 86 slides while pressing onto the inner wall 78 of the blood collecting tube 12 by means of as elastic force so as to control the sinking speed of the member main body 83 in the serum 6, and the member main body 83 contains the clot 7 without causing any disturbances. As a result, the member main body 83 is stopped at the boundary between the serum 6 and the clot 7 to prevent clot 7 from moving into the serum 6.

In exactly the same manner as with the blood separating members 41 and 71 of the first and second embodiments, this blood separating member 81 also is able to control the sinking speed of the member main body 83 in the serum 6, so that the member main body 83 is stopped at the boundary between the clot 7 and the serum 6 to prevent the clot 7 from moving into the serum 6.

[Fourth Embodiment]

Fig. 9 is a vertical section view showing a blood separating member (component separating member) 91 according to a fourth embodiment of the present invention. The roof plate 54 and thin leaf 77 on the member main body 73 of the blood separating member 71 according to the second embodiment has been curved into a concave shape, and the neck portion 93 of the roof plate 54 is made shorter.

In the first through third embodiments, the elastic member 45 and thin leaf 77 are flat plates which extend horizontally outward. However, when these are made into flat plates, the elastic member 45 and thin leaf 77 change shape in a complicated manner, not uniformly, when contacting the inner walls of the blood collecting tubes 42 and 12. Therefore, when sinking through the serum 6 during centrifugal separation, the elastic member 45 and the thin leaf 77 do not change shape easily, sometimes making sinking impossible.

Therefore, by reducing the gap between the thin leaf 77 and the top surface of the member main body 73 of the blood separating member 71, curving the top surface 92 of the thin leaf 77 and the member main body 73 into a concave shape, and making the diameter of the neck portion 93 smaller, the thin leaf 77 is made to change shape uniformly when contacting the inner surface of the blood collecting tubes 42 and 12.

For example, if the blood collecting tube 12 is plastic, then the surface is usually tapered so that the diameter is slightly reduced from the mouth portion 12a to the bottom portion as shown in Fig. 10, so that the boundary between the clot 7 and the serum 6 has a circular horizontal cross-section or an approximate elliptical cross-section when slightly inclined. At this time, the circumference of the ellipse is larger than the circumference of the circle.

In this case, taking the radius of the circle to be a, the minor radius of the ellipse to be a, and the major radius to be b, then the circumferences can be represented as:

$$\text{Circumference of Ellipse: } L1 \approx \pi\,(a+b) \approx 3.14 \times (a+b)$$

$$\text{Circumference of Circle: } L2 \approx \pi\,(2 \times a) \approx 3.14 \times 2 \times a$$

In this case, from the fact that $a < b$, it follows that $L2 < L1$.

In order to make the thin leaf 77 contact the blood collecting tube 12 without any gaps at the boundary surface between the clot 7 and the serum 6, the circumference of the thin leaf 77 when the shape is changed to an ellipse by slightly inclining the curved thin leaf 77 should be made to match the circumference of the inclined cross-sectional surface of the blood collecting tube 12. In order to make this phenomenon possible, the thin leaf 77 is curved and the neck portion 93 is made shorter to make it easier for the thin leaf 77 to incline.

While the relationship between the diameter of the thin leaf 77 and the diameter of the neck portion 93 depends upon the thickness of the thin leaf 77, the diameter of the neck portion 93 should preferably be approximately 25 % ~ 45 % of the diameter of the thin leaf 77.

The reason for the range of 25 % ~ 45 % is that at less than 25 %, there is the risk of the needle hitting the inner wall of the space portion 53 when inserting the blood collecting needle, and at more than 45 %, it becomes difficult for the thin leaf 77 to be able to tilt, so that it becomes difficult to sink through the serum 6 during centrifugal separation in a blood collecting tube 12 having a tapered surface.

Aside from being circular, the horizontal cross-sectional shape of the neck portion 93 can be made rectangular in order to make it easier for the thin leaf 77 to tilt.

If the degree of tapering at one side of the inner diameter of the blood collecting tube 12 is 5/1000 or less, then a reliable sealing effect can be obtained at the position of the boundary between the serum 6 and the clot 7.

As with the blood separating member 71 according to the second embodiment, this blood separating member 91 is also able to control the sinking speed of the member main body 73 in the serum 6, and to stop the member main body 73 at the boundary between the clot 7 and serum 6. Furthermore, the sealing effect improves because the roof plate 54 of the member main body 73 and the top surface 92 of the thin leaf 77 are curved, and the neck portion 93 of the roof plate 54 is made shorter, so that the clot 7 and serum 6 can be reliably separated and mutual shifting can be reliably prevented.

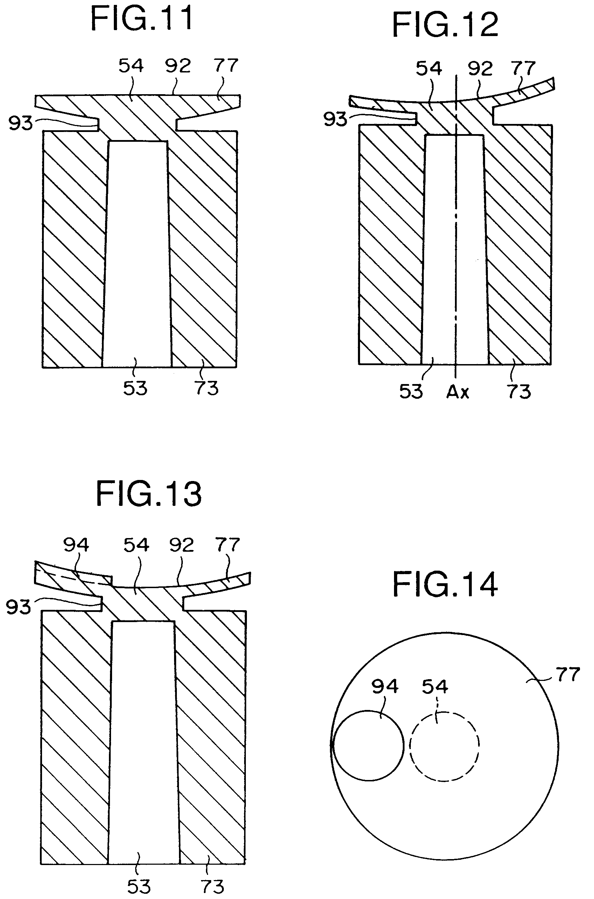

While the roof plate 54 of the member main body 73 and the top surface 92 of the thin leaf 77 are curved into concave surface in the blood separating member 91 according to the present embodiment, the bottom surface of the thin leaf 77 can be curved into a concave surface while leaving the top surface 92 flat, as shown in Fig. 11.

Additionally, as shown in Fig. 12, the thin leaf 77 can be slightly inclined with respect to the axis Ax of the member main body 73, so that one end of the curved top surface 92 flares upward. In this case, blood is able to flow smoothly into the curved top surface of the thin leaf 77, so as to reduce the resistance when introducing the member main body 73 into the blood, thereby allowing the member main body 73 to sink through the serum without jerking to make the member main body 73 into a good partition for separating the clot 7 and the serum 6.

Additionally, as shown in Figs. 13 and 14, a disc-shaped protruding portion 94 having a diameter less than the width of the thin leaf 77 can be unitarily provided on the thin leaf 77. In this case, the member main body 73 can be smoothly introduced into the blood, thereby making the member main body 73 into an excellent partition for separating the clot 7 and serum 6.

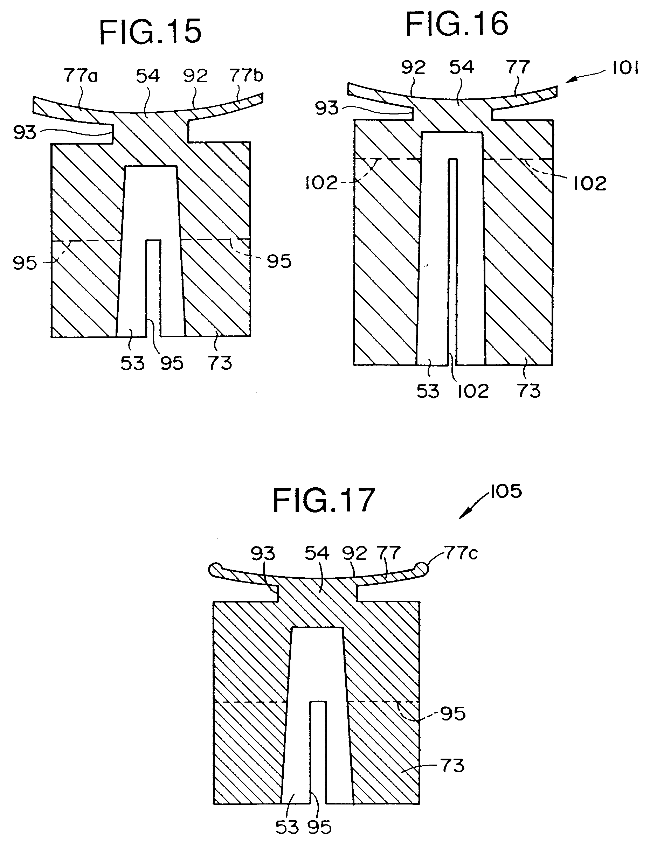

Furthermore, as shown in Fig. 15, one side 77a of the thin leaf 77 can be made thicker than the other side 77b as shown in Fig. 15. In this case, after sinking due to the centrifugal force during centrifugal separation and reaching the top surface of the serum 6, it is able to smoothly enter the serum 6. Furthermore, open-bottomed slits 95 having lengths approximately half that of the height of the member main body 73 can be made to pass through the member main body 73 can be formed at three positions on the member main body 73 symmetric with respect to the axis Ax.

By forming the slits 95, the air in the space portion 53 can be removed from under the upper end of the slits 95 during centrifugal separation. As a result, the amount of air held inside the space portion can be arbitrarily set by arbitrarily changing the lengths of the slits 95.

As a result, the fluctuation width for the apparent specific gravity of the member during centrifugal separation can be controlled, so that the sinking speed of the member in serum can be arbitrarily set to arbitrarily control the sinking speed.

[Fifth Embodiment]

Fig. 16 is a vertical section view showing a blood separating member (component separating member) according to a fifth embodiment of the present invention. Open-bottomed slits 102 which reach close to the top portion of the space portion 53 are made to pass through the member main body 73 are formed at three positions on the member main body 73 symmetric with respect to the axis Ax on the blood separating member 91 according to the fourth embodiment.

Hereinbelow, experimental examples and comparative examples which were performed to clarify the effects of the blood separating member 101 will be explained.

(Experimental Example)

A blood separating member 101 having a unitary roof plate 54, thin leaf 77 and member main body 73 was formed from an elastomer having a specific gravity of 1.09 and a rubber hardness of 45 degrees (JIS K-6301). The member main body 73 of this blood separating member 101 had a height of 18 mm and an outer diameter of 12.9 mm, and had air escape slits 102 are formed 2.5 mm lower than the top end of the space portion 53, and the slits 102 having widths of 1.5 mm formed at three locations symmetric with respect to the axis Ax of the member main body 73.

The thin leaf 77 had an outer diameter of 13. 6 mm and a thickness of 0.75 mm, while the radius of curvature of the curved roof plate 54 and top surface 92 of the thin leaf 77 was 23.6 mm. Additionally, the diameter of the neck portion 93 was 5 mm.

Furthermore, the blood collecting tube 12 was injection molded from polyethylene terephthalate (PET) to have an effective volume of 10 ml, an inner diameter at the top end of 13. 4 mm, and the inner surface was tapered to an incline of 3/1000 on one side.

The inner diameter at the position of the boundary between the clot and serum in the blood collecting tube 12 was 13. 15 mm when assuming the volume of blood injected to be 9 ml, the proportion of serum in the whole blood to be 55 V/V%, and the boundary surface to be horizontal; when assuming the volume of injected blood to be 3 ml and the proportion of serum in the whole blood to be 55 V/V%, then the diameter was 13.10 mm.

Next, two of these blood collecting tubes 12 were prepared, after which a short tube was inserted into one of the blood collecting tubes 12 to the position of the boundary between the clot and serum for 9 ml of blood and a short tube was inserted into the other blood collecting tube 12 to the position of the boundary between the clot and serum for 3 ml of blood, then the one blood collecting tube 12 was injected with 9 ml and the other blood collecting tube was injected with 3 ml of bovine fetal serum as a replacement for blood.

Subsequently, blood separating members 101 were put into each of these blood collecting tubes 12, then centrifugally separated for five minutes at 1000 g, whereupon the blood separating members 101 sinked into the serum in both blood collecting tubes 12, and were stopped on top of the short tubes which act as stoppers at the positions of the boundaries between the clot and serum. Additionally, the thin leaves 77 and the inner surfaces of the blood collecting tubes 12 contacted in such a way that the entire circumference of the thin leaves 77 completely contacted the inner surfaces of the blood collecting tubes 12 with the thin leaves 77 slightly tilted.

(Comparative Example)

A member main body having a shape absolutely identical to that of the member main body 73 of the above embodiment, aside from the top surface 92 of the thin leaf 77 and the roof plate 54 being flat, was formed and the same experiment as described above was conducted using this member main body. As a result, it was observed that the member main body did not sink to the short tube which acts as a stopper at the position of the boundary between the clot and the serum, stopping halfway, or stopping above the serum. Additionally, the contact between the thin leaf 77 and the inner surface of the blood collecting tube 12 was such that the thin leaf 77 contacted the inner surface of the blood collecting tube 12 in a warped state, so that the contact between the entire circumference of the thin leaf 77 and the inner surface of the blood collecting tube 12 was incomplete.

In this way, when the top surface of the thin leaf 77 and the roof plate 54 were made flat in a member main body, warping occurred around the thin leaf 77 when contacting a blood collecting tube 12 having a diameter less than the diameter of the thin leaf 77, so as to generate non-reproducible deformations and present difficulties in the reliability as a sealing material.

With this blood separating member 101 as well, the sealing effect is improved, and the clot 7 and serum 6 can be reliably separated while reliably preventing their mutual shifting.

[Sixth Embodiment]

Fig. 17 is a vertical section view showing a blood separating member (component separating member) 105 according to a sixth embodiment of the present invention. The thickness of the fringe portion around the entire circumference of the thin leaf 77 of the blood separating member according to the fourth embodiment is made greater than the thickness of the thin leaf, so as to form a thick portion 77c having an approximately circular cross-section.

With this blood separating member 105, the motion of the thin leaf 7 is made more flexible and a seal with the inner wall of the blood collecting tube 12 is ensured by reducing the thickness of the thin leaf 77, and the contact area with the inner wall of the blood collecting tube is increased without increasing the frictional resistance by making the fringe portion of the thin leaf 77 into a thick portion 77c.

With this blood separating member 105, the thick portion 77c is deformed into a roughly elliptical shape when sinking through the serum due to centrifugal separation, so as to accurately maintain contact with the inner wall of the blood collecting tube 12. Additionally, this thick portion 77c makes movement easier when contacting the inner wall of the blood collecting tube 12 in a roughly elliptical shape.

With this blood separating member 105 as well, the same effects as with the blood separating member 91 according to the fourth embodiment can be achieved.

Furthermore, the fringe portion of the thin leaf 77 is made into a thick portion 77c having a roughly circular cross-section, so that the contact surface with the inner wall of the blood collecting tube 12 can be increased without increasing the frictional resistance, and making movement easier even when contacting the inner wall of the blood collecting tube 12.

While the fringe portion of the thin leaf 77 has been made into a thick portion 77c having a roughly circular cross-section in the present embodiment, it is only necessary that the fringe portion of the thin leaf 77 be thicker than the central portion, so that the cross-sectional shape is not restricted to being roughly circular and may have various cross-sectional shapes.

In this way, the blood separating members of the above-mentioned first through sixth embodiments are able to form a partition at the boundary between the serum 6 and clot 7 while maintaining air in all or part of the space portion 53 when performing centrifugal separation. The advantage of these blood separating members is in the cushioning effect of the air within the space portion 53, which controls the sinking speed of the blood separating member in the serum during centrifugal separation, and weakens the force of impact on the clot 7.

For example, in a centrifugal separation of 9 ml of blood at a centrifugal force of 1000 g, the air inside the space portion 53 is compressed by the centrifugal force after the centrifugation operation is started and the blood separating member reaches the top surface of the serum. When slits or through holes are formed to allow air to escape from the space portion 53, the air underneath these slits or through holes can pass through to the outside, but the air above these is trapped in the space portion 53 without escaping, and is compressed by the centrifugal force.

In this case, when the apparent specific gravity of the blood separating member in consideration of the volume (the weight is ignored) of the compressed air is greater than that of the serum, the blood separating member overcomes buoyancy to sink into the serum. Once inside the serum, the blood separating member sinks through the serum, but as the blood separating member drops the centrifugal force increases, and the compression ratio of air in the space portion 53 of the blood separating member is increased while the volume is decreased, so that the apparent specific gravity of the blood separating member in consideration of the volume of air becomes greater. As a result, the buoyancy of the blood separating member is overcome so as to increase the sinking speed in the serum.

The through holes 87 or slits 95 are formed to decrease the amount of air held by the space portion 53 in order to suppress the fluctuation width of the apparent specific gravity of the blood separating member during centrifugal separation, thereby broadening the range of control for the sinking speed of the member in serum.

As explained above, with the blood separating members according to the first through sixth embodiments, the sinking speed of these blood separating members directly after immersion in the serum can be suppressed by holding part or all of the air inside the space portion 53. As a result, centrifugal separation can be performed without the blood cell which are suspended in the serum escaping above the blood separating member immediately after centrifugation, thereby decreasing the number of blood cells on top of the blood separating member.

With regard to these blood separating members, their actual specific gravities need only be greater than that of serum and can be greater than that of clot, due to the air held in their space portions 53.

Additionally, the thin leaves 77 must be elastic and have plasticity.

The reason for this is that the centrifugation operation is continued until the designated time has passed, even after the blood separating member has reached the boundary between the serum and the clot. At this time, the peripheral portion of the thin leaf 77 attempts to enter between the blood collecting tube and the thin leaf 77 due to the weight of the thin leaf 77 itself, so as to form a stronger partition. Additionally, when the centrifugation operation has been halted, the stronger partition formed during centrifugation is continually held in that state due to the thin leaf 77 having both elasticity and plasticity.

The elastomer used in the experimental example was a polystyrene type thermoplastic elastomer, mixed from an elastomer with a high molecular weight and an elastomer with a low molecular weight to give it elasticity such as not to be deformed during work such as when moving the part, or applying to the blood collecting tube, while being soft enough to plastically deform to form a partition during centrifugal separation.

The elastomer used in the experimental example had a specific gravity of 1.09, a rubber hardness of 45 degrees, and an elasticity coefficient of 900 kPa.

Among thermoplastic elastomers of the same type, a thermoplastic elastomer having a high molecular weight is mixed with a thermoplastic elastomer having a low molecular weight to form a thermoplastic elastomer with an elasticity coefficient of 1 Mpa or less, and the blood separating member is molded using this elastomer. As a result, the tightness of contact of the blood separating member on the inner wall of the blood collecting tube 12 after centrifugal separation is improved so as to ensure a more reliable seal.

[Seventh Embodiment]

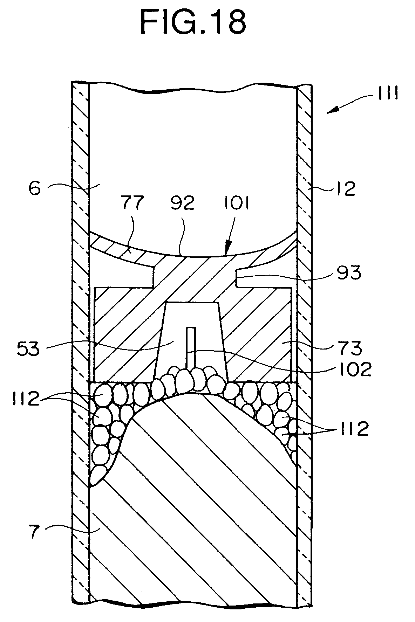

Fig. 18 is a vertical section view showing a blood separating tube (component separating tube) 111 according to a seventh embodiment of the present invention, wherein a plurality of pellets (grains) 112, 112, . . . are inserted into a blood collecting tube 12 along with a blood separating member according to the fifth embodiment.

As pellets 112, it is preferable that the pellets have grain sizes in the range of 0.1 ~ 2.0 mm, with a specific gravity less than the specific gravity of clot 7, preferably a specific gravity of between those of serum 6 and clot 7, and that the material be inert with respect to serum 6 and clot 7. For example, pellets composed of plastics such as polystyrene and ABS resin, or glass beads which have been adjusted for their specific gravity by containing bubbles.

The specific gravities of the pellets 112 and the blood separating member 101 do not each necessarily have to be between those of serum and clot, so that the specific gravity of the pellets 112 can be less than the specific gravity of clot and the specific gravity of the blood separating member 101 can be greater than the specific gravity of serum.

Additionally, a mixture wherein a blood coagulant has been dispersed in a binder which is water-soluble and inert with respect to serum may be adhered to part or all of the surfaces of the pellets 112. Furthermore, as for the types of these pellets 112, there can be one type, or two or more types.

With the blood separating members according to the first through sixth embodiments described above, the following types of phenomena can occur at times.

(1) Blood cells can ride on top of the member main body after centrifugal separation.
(2) Since the member main body directly contacts the top surface of the clot layer during centrifugal separation, there is the risk of destroying blood cell due to the impact.
(3) In order to ensure the stability as a partition, the height of the member main body should be made tall; a height of 15 ~ 20 mm is necessary. Therefore, the increase in volume reduces the effective capacity of the blood collecting tube, which results in cost increases. Furthermore, the molding of the member main body can become difficult.
(4) When the member main body remains as a partition at the boundary between the serum and clot after centrifugation, serum occasionally exists inside the clot layer. This fact especially tends to reduce the recovery rate of serum.

This blood separating tube 111 improves upon the drawbacks occurring when the blood separating members of the first through sixth embodiments are used.

Blood is separated using this blood separating tube 111 by inserting a designated number of pellets 112 into the blood collecting tube 12, injecting collected blood into the blood collecting tube 12, allowing the blood to coagulate after storing the blood collecting tube 12 indoors, and inserting the blood separating member 101 into the blood collecting tube 12. The blood separating member 101 can be pre-attached to a blood collecting tube 12 into which pellets 12 have been inserted, or can be inserted within the blood collecting tube 12 immediately before the centrifugal separating operation. Additionally, the amount of pellets is such as to allow a partition to be formed to some extent from only the pellets 112, and must be an amount sufficient to enter the clot 7 to push the serum 6 within the clot 7 above the blood separating member 91; specifically, 0.2 ~ 0.6 g is preferable.

Hereinbelow, an experimental example conducted to clarify the effects of the blood separating tube 111 will be explained.

(Experimental Example 1)

A blood separating member 101 was molded from an elastomer with a specific gravity of 1.09 and a rubber hardness of 45 degrees (JIS K-6301). The height of the member main body 73 of this blood separating member 101 was 18 mm (and 9 mm), the outer diameter was 12.9 mm, and air escape slits 102 having widths of 1.5 mm were formed at three positions on the member main body 73 2.5 mm below the top end of the space portion 53.

The thin leaf 77 had an outer diameter of 13.6 mm and a thickness of 0.75 mm, and the radius of curvature of the curved top surface 92 was 23.6 mm.

Additionally, the diameter of the neck portion was 5 mm. Furthermore, the blood collecting tube 12 was injection molded from polyethylene terephthalate (PET) such that the inner surface was tapered to an incline of 3/1000 to one side, the inner diameter of the top end was 13.4 mm, and the effective volume was 10 ml.

Next, three of these blood collecting tubes 12 were prepared; the first blood collecting tube 12 had only a blood separating member 101, the second blood collecting tube 12 had a blood separating member 101 having a height of 18 mm and 0.6 g of pellets 112 composed of polystyrene grains having grain sizes of 0.2 ~ 1.7 mm, and the third blood collecting tube had a blood separating member 101 having a height of 9 mm and 0.6 g of pellets 112 composed of polystyrene grains (PS grains) having grain sizes of 0.2 ~ 1.7 mm.

The coagulation in the first blood collecting tube 12 was promoted by a coagulation promotion procedure used in commercially available blood collecting tubes, that is, by coating the inner wall of the blood collecting tube 12 with a mixture of polyvinyl pyrrolidone (PVP) and silica grains.

In the second blood collecting tube 12, among the 0.6 g of PS grains, 0.2 g were treated with a coagulation acceleration procedure. That is, a blood coagulation promoting agent dispersed in water-soluble polyethylene glycol was adhered to the powdered silica microparticles and PS grains with a grain size of 0.2 ~ 0.4 mm. Grains having a grain size of 0.8 ~ 1.7 mm were used for the remaining 0.4 g of PS grains.

6 ml of blood was injected into each of these first through third blood collecting tubes 12, and these were allowed to stand for 60 minutes to coagulate the blood. Then, they were centrifugally separated for five minutes as 1500 g.

As a result of the centrifugal separation, the blood separating member 101 was positioned at the boundary between the clot and serum to form a partition in the first blood collecting tube 12, but a few blood cells remained on the top surface 92. Additionally, some serum remained below the blood separating member 101.

On the other hand, while the second and third blood collecting tubes 12 exhibited results similar to the first blood collecting tube 12 in that the blood separating member 101 was positioned at the boundary between the clot and serum, they had no blood cell on the tope surfaces 92 and there was no serum left in the clot layers below the blood separating members 101. Additionally, the pellets were concentrated beneath the blood separating members 101, so as to fill the gaps between the clot and the inner wall of the blood collecting tube 12.

In this way, by putting at least a designated amount of pellets 112 beneath the blood separating member 101, blood cell can be prevented from coming above the blood separating member 101, and the serum recovery rate can be increased by removing the serum from the gaps between the inner wall of the blood collecting tube 12 and the clot layer beneath the blood separating member 101.

(Experimental Example 2)

This Experimental Example 2 is absolutely identical to Experimental Example 1, except that the amount of blood is 3 ml.

As a result of centrifugal separation, in the first blood collecting tube 12, there was no blood cell on the top surface 92 of the blood separating member 101, but the serum had turned slightly brownish and it was apparent that hemolysis had occurred. Additionally, some serum remained in the clot layer below the blood separating member 101.

On the other hand, in the second and third blood collecting tubes 12, there was no blood cell on the top surface 92 of the blood separating member 101, and no serum was found in the clot layer underneath the blood separating member 101.

In this way, by putting at least a designated amount of pellets 112 beneath the blood separating member 101, the force whereby the blood separating member 101 crushes the clot layer can be converted into a force for pushing the surface of the pellets 112, thereby preventing hemolysis due to blood cell destruction.

With this blood separating tube 111, the pellets 112 themselves partially function as a partition, so that by putting at least a designated amount of pellets 112 beneath the blood separating member 101, the probability for the existence of blood cell on the top surface 92 of the blood separating member 101 after centrifugal separation can be made extremely small.

Additionally, the blood separating member 101 sinks and hits the partition formed by the pellets 112 gathering at the boundary between the clot 7 and serum 6 during centrifugal separation, so as to reduce the impact on the clot layer.

Additionally, the pellets 112 form a part of the partition, so that the height of the blood separating member 101 can be small and the structure can be simple, thereby reducing costs and making molding easier.

Additionally, the amount of serum in the gap between the blood separating member 101 and the inner wall of the blood collecting tube 12 can be reduced because the height of the blood separating member 101 can be reduced.

Additionally, even if the clot layer below the blood separating member 101 does not completely fill the blood collecting tube 12 with clot, the serum is removed when the pellets 112 enter the clot layer so as to increase the serum recovery rate.

[Eighth Embodiment]

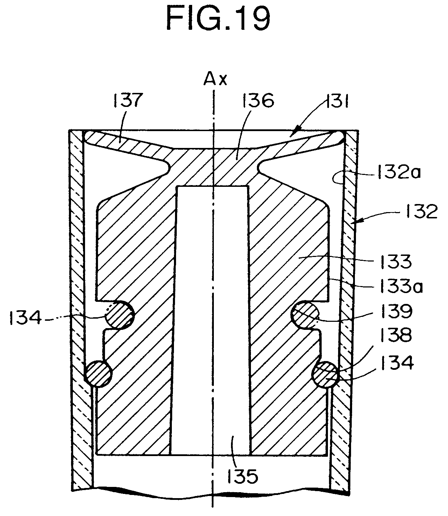

Fig. 19 is a vertical section view showing a blood separating tube (component separating tube) according to an eighth embodiment of the present invention. In the drawing, reference numeral 131 denotes a blood separating member, and reference numeral 132 denotes a cylindrical blood collecting tube (cylindrical container) wherein the diameter of the mouth portion 132a has been slightly enlarged, into which the blood separating member 131 has been inserted.

The blood separating member 131 comprises a cylindrical member main body 133 and an O-ring (elastic member) 134 having a circular cross-section.

The member main body 133 is elastic and roughly cylindrical, with a top portion which is conical. A space portion 135 which opens downward is formed coaxially at the central portion, the roof plate 136 above this space portion 135 being thinned in the vertical direction so as to allow a blood collecting needle to pass easily through, and the peripheral portion thereof having a diameter enlarged in the horizontal outward direction which has a slight upward curve, thus forming a thin leaf 137 which is thinner and larger in diameter than the member main body 133. Additionally, a first groove 138 having a semicircular cross-section with a maximum depth approximately equal to the radius of the cross-section of the O-ring 134 is formed at the outer circumferential portion 133a, and a second groove 139 having a maximum depth approximately equal to the diameter of the cross-section of the O-ring 134 is formed at a position above the first groove 138, each making a single circuit in the circumferential direction.

Here, the outer diameter of the member main body 133 between the first groove 138 and the second groove 139 is designed to be slightly smaller, so that the O-ring 134 can easily move in the direction of the axis Ax.

This O-ring is held by being fitted into the first groove 138, and the elastic force presses it towards the inner wall of the mouth portion 132a of the blood collecting tube 132. The magnitude of this pressing force is absolutely uninfluenced by the force required to pass the blood collecting needle through, but the shape, dimensions, and material of the O-ring 134 and member main body 133 is set such that during the centrifugation operation, the member main body 133 drops when the centrifugal force reaches a value less than the set centrifugal force, for example a centrifugal force of approximately 800 g for a set centrifugal force of approximately 1000 g.

Additionally, the diameter of the hole for housing the O-ring 134 is set so as to allow it to be housed within the second groove 139 of the member main body 133 with a slight expansive force.

The blood separating member 131 should be such that the average specific gravity of the O-ring 134 and the member main body 133 in consideration of the air inside the space portion 135 is between those of serum and clot, should allow a blood collecting needle to pass easily through, and should be soft and elastic so as to close up after the blood collecting needle has been removed. The average specific gravity of the O-ring 134 and the member main body 133 without consideration of the air inside the space portion 135 is not restricted to being between those of serum and clot, and for example can be greater than that of clot.

As for the materials, rubbers or elastomers such as styrene/butadiene rubber, butyl rubber, silicone rubber, polystyrene type elastomers, polyamide type elastomers, or silicone type elastomers, or mixtures of these materials with inorganic materials such as barium sulfate to adjust the specific gravity are preferable for use. The hardnesses of the rubbers and elastomers should be 30 ~ 60 degrees according to the JISA (JISK6301).

Next, methods for separating blood components using this blood separating member 131 will be explained.

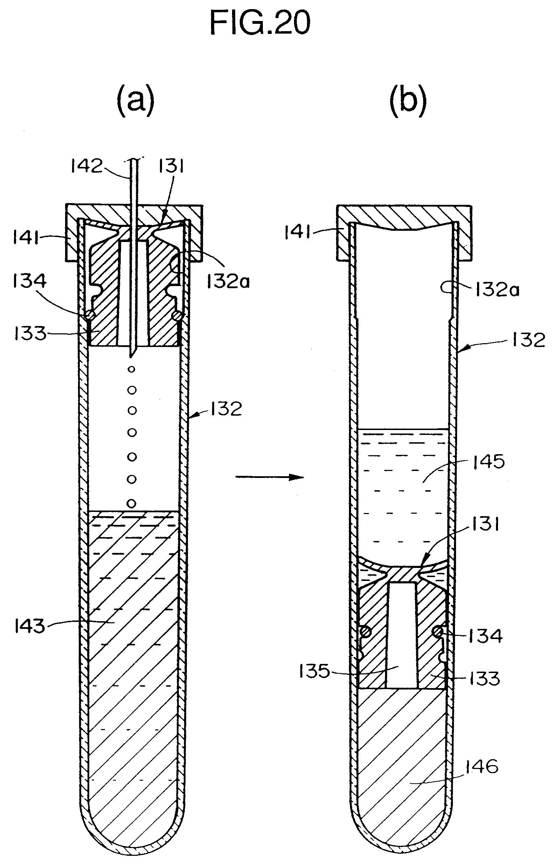

Here, the pre-insertion format will be explained with reference to Fig. 20.

First, as shown in Fig. 20A, a blood separating member 131 is put on the mouth portion 132a of a blood collecting tube 132, and a cap 141 is attached. In this case, the member main body 133 is pushed downward by the cap 141, but the O-ring 134 fitted into the first groove 138 keeps the member main body 133 from slipping further into the blood collecting tube 132 and stops it at the upper portion of the blood collecting tube 132. The areas above and below the member main body 133 are completely partitioned. The blood collecting tube 132 is a vacuum blood collecting tube having an interior which is held to about 150 mmHg.

Next, the cap 141 and the blood separating member 131 are punctured from above by a blood collecting needle 142, and collected blood (fluid sample) 143 is injected. While the amount of blood injected is determined by the degree of depressurization in the blood collecting tube 132, it is always positioned below the member main body 133, and does not go above it.

Next, the blood is coagulatd by leaving the blood collecting tube 132 at indoors.

Then, a centrifugal separation is performed as shown in Fig. 20B. After the centrifugal separation operation has started and the centrifugal force exceeds a designated value (in this case, approximately 800 g), the member main body 133 drops due to the centrifugal force and shifts slightly downward, whereby the O-ring which is pressed against the inner wall of the mouth portion 132a is released from the first groove 138. When the member main body 133 drops further due to the centrifugal force, the second groove 139 moves to the position of the O-ring 134 to house it.

When housed, the O-ring 134 no longer presses against the inner wall of the blood collecting tube 132, so that there is no force opposing the sinking of the member main body 133 other than the pressing force of the thin leaf 137, allowing it to sink gradually while maintaining good horizontal balance, and moving it to the boundary between the serum 145 and the clot 146 to press against the clot 146. At this point, the elastic force of the thin leaf 137 presses it against the inner wall of the blood collecting tube 132. Additionally, the mutual movement of the member main body 133 and the clot 146 during centrifugal separation becomes smooth because the air inside the space portion 135 acts as a cushion.

As explained above, the member main body 133 moves to the boundary between the serum 145 and the clot 146 to press against the clot 146, thereby preventing the clot 146 from moving into the serum layer 145. Therefore, the serum 145 and clot 146 are completely separated by this blood separating member 131, so that the blood cells in the clot 146 are not able to mix into the serum 145. At the same time, the clot are not destroyed because the impact during centrifugal separation is reduced. Furthermore, when the inner diameter of the blood collecting tube 132 changes, the thin leaf 137 presses against the inner wall of the blood collecting tube 132, so that the serum 145 and the clot 146 are able to be completely separated.

Next, the post-insertion format will be explained.

Collected blood 143 is injected into the blood collecting tube 132, and the blood is coagulated by leaving the blood collecting tube 132 indoors. Then, a blood separating member 131 is put onto the mouth portion 132a of the blood collecting tube 132 and the cap 141 is applied.

Thereafter, the centrifugal separation operation is absolutely identical to that of the above-mentioned pre-insertion format.

In this format also, the member main body 133 moves to the boundary between the serum 145 and the clot 146 to press against the clot 146, thereby preventing clot 146 from moving into the serum 145 layer, as a result of which the serum 145 and the clot 146 are completely separated by this blood separating member 131 to eliminate the problem of blood cells from the clot 146 mixing into the serum 145.

Hereinbelow, an experimental example performed to clarify the effect of the blood separating member 131 will be explained.

Barium sulfate was added to a commercially available elastomer to adjust the specific gravity, and an inactive oil was added to adjust the hardness, resulting in an elastomer having a specific gravity of 1.09 and a hardness of 30 (JISK6301). This elastomer was molded to obtain a desired member main body 133.

This member main body 133 had a height of 18 mm, an outer diameter of 12.9 mm, a first groove diameter of 11.0 mm, a second groove diameter of 9.0 mm, a diameter between the first groove 138 and the second groove 139 of 12.0 mm, and the thin leaf had an outer diameter of 13.6 mm, a thickness of 0.75 mm with a slight upward curvature. The roof plate 136 had a thickness of 2.0 mm and a diameter of 5 mm. Additionally, the volume of the member main body was 1.65 cm$^3$, the volume of the space portion 135 was 0.13 cm$^3$, and the weight was 1.8 g.

On the other hand, the O-ring 134 was molded from natural rubber having a specific gravity of 0,93 and a hardness of 60, forming a ring having an inner diameter of 8.5 mm, an outer diameter of 11.82 mm, and a diameter of circular cross secton of 1.66 mm.

When this O-ring 134 was fitted into the first groove 138, the outer diameter became 14 mm, and when fitted into the second groove 139, the outer diameter became 12 mm.

Additionally, the blood collecting tube 132 was injection molded from polyethylene terephthalate (PET) to a shape such as to have an effective capacity of 10 ml, a height of 100 mm, an inner diameter of 13.4 mm at the top portion, an inner diameter of 14.6 mm from the top end of the mouth portion 132a to a depth of 14 mm, and an inner diameter of 13.10 mm at the bottom portion.

The O-ring 134 was fitted into the first groove 138 to form the blood separating member 131, and after inserting this into the mouth portion 132a of the blood collecting tube 132, the member main body 133 was affixed to the mouth portion 132a by the O-ring 134. It was discovered that when this member main body 133 is firmly pressed by one's finger, the O-ring 134 moves from the first groove 138 to the second groove 139, and the member main body 133 slowly drops through the blood collecting tube 132.

Next, a blood coagulation promoting agent was coated onto the inside of this blood collecting tube, 6 ml of fresh blood was injected and left for 60 minutes to allow sufficient coagulation. Next, this blood separating member 131 was inserted into the mouth portion 132a of the blood collecting tube 132, and a centrifugal separation was performed for five minutes at 1000 g. As a result, the blood separating member 131 was positioned at the boundary between the serum 145 and the clot 146, and it was found that there was no blood cell above the member main body 133 and the clot 146 would not move through the member main body 133 to the serum 145 side even if the blood collecting tube 132 was inverted. At this time, the O-ring 134 had moved from the first groove 138 to the second groove 139.

Additionally, upon measuring the potassium concentration inside the serum 145 after centrifugal separation, it was found to be 4.3 mEq/l immediately after centrifugal separation, 4.4 mEq/l after seven days of storage at 4 °C, indicating that the blood separating member 131 had completely separated the serum 145 and the clot 146, and that this was stable over time as well.

As explained above, this blood separating member 131 comprises a tubular member main body 133 and an O-ring 134, wherein the peripheral portion of the roof plate 136 of the member main body 133 is composed of a thin leaf 137 which is larger in diameter and thinner than the member main body 133, a first groove 138 and a second groove 139 at a position above the first groove 138 are formed around the entire circumference at the outer circumferential portion 133a of the main body 133, with the O-ring 134 being supported by fitting into the first groove 138 and being pressed against the inner wall of the mouth portion 132a of the blood collecting tube 132 by the elastic force, so as to ensure that a complete partition is formed between the areas above and below the member 131 before centrifugal separation. Additionally, the blood separating member 131 can be prevented from adhering to the wall surfaces of the blood collecting tube 132 even when letting rest for long period of time. As a result, more accurate analysis values are able to be determined.

Additionally, when the inner diameter of the blood collecting tube 132 fluctuates, the thin leaf 137 presses against the inner wall of the blood collecting tube 132, so that the serum 145 and clot 146 are able to be completely separated, and the blood cells in the clot 146 can be prevented from mixing into the serum 145.

Furthermore, when the blood separating member 131 is left to stay in the blood collecting tube 132 for a long period of time after separation, the thin leaf 137 continues to isolate the serum 145 and the clot 146, so as to prevent the blood platelets in the clot 146 from mixing into the serum 145.

Additionally, a space portion 135 which opens downward is formed coaxially at the central portion of the member main body 133, so that the air filling the space portion 135 is compressed by the pressure generated by the centrifugal force and held in a state of entrapment in the space portion 135, as a result of which the partitioning capabilities of the roof plate 136 are increased and the shifting of components can be completely prevented by the air layer, even if there are needle holes from the blood collecting needle 142 in the roof plate 136. Therefore, the shifting of the components of serum 145 and clot 146 can be completely cut off. Additionally, the air inside the space portion 135 acts as a cushion to reduce the impact on the clot 146.

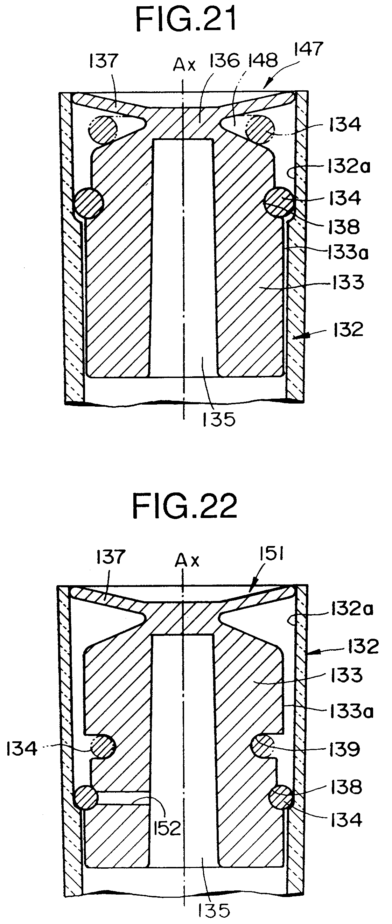

Additionally, while a first groove 138 and a second groove 139 are formed around the entire circumference at the outer circumferential portion 133a of the member main body 133, as an alternative the first groove 138 can be formed around the entire circumference at an upper portion of the outer circumferential portion 133a of the member main body 133, and the gap 148 formed between the upper portion of the member main body 133 and the thin leaf 137 can be made into the second groove, as with the blood separating member 147 shown in Fig. 21.

In this case, the O-ring 134 is supported at a position in the upper portion of the outer circumferential portion 133a, so that the depth of the mouth portion 132a can be set so that the bottom end of the mouth portion 132a of the blood collecting tube 132 aligns with the position of the O-ring 134.

[Ninth Embodiment]

Fig. 22 is a vertical section view showing a blood separating member 151 according to a ninth embodiment of the present invention. In the drawing, reference numeral 152 denotes through holes formed in the member main body 33 to connect the first groove 138 with the space portion 135; the other features are exactly identical to those of the blood separating member 131 described above, so their explanations will be omitted.

With this blood separating member 151, the through holes 152 are sealed off by the O-rings 134 and the areas above and below the blood separating member 151 are completely partitioned until centrifugal separation is performed, so that there is no risk of the collected blood escaping above the member 133.

When centrifugal separation is performed, the air below the through holes 152 in the space portion 135 move upward through the first groove 138 during centrifugal separation, so that the air inside the space portion 135 below the through holes 152 can be removed.

Additionally, the through holes 152 are formed in radial directions on the member main body 133, so that the amount of air held can be decreased and the fluctuations in the specific gravity of the blood separating member 151 during centrifugation can be reduced.

Additionally, if the passage between the areas above and below the member main body 133 must be opened prior to the centrifugal separation operation and the areas above and below the member main body 133 must be partitioned immediately after the centrifugation is started, then the through holes should be formed so as to connect the second groove 139 with the space portion 135.

[Tenth Embodiment]

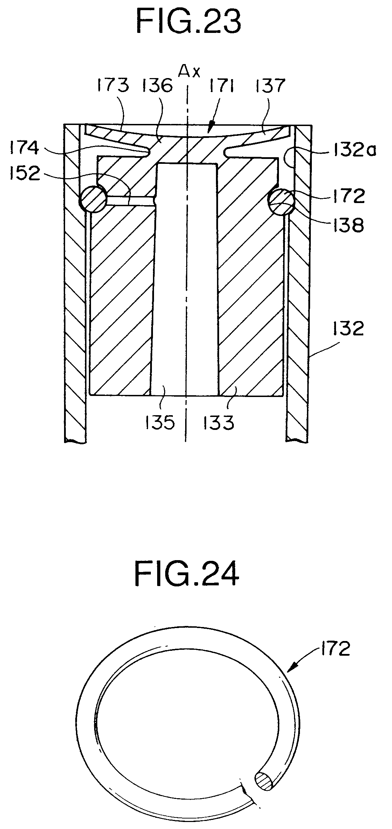

Fig. 23 is a vertical section view showing a blood separating member 171 according to a tenth embodiment of the present invention. In the drawing, reference numeral 172 denotes an elliptical O-ring.

In this blood separating member 171, the roof plate 136 of the member main body 133 and the top surface 173 of the thin leaf 137 are curved into a concave shape, and the neck portion 174 of the roof plate 136 is shortened.

The elliptical O-ring 172 is composed of a plastic such as polyethylene, polypropylene or the like, having the overall shape of an elliptical ring and a circular cross-section as shown in Fig. 24, wherein the inner circumference is set to be approximately equal to the circumference of the groove 138, and the outer circumference is set to be slightly less than the inner diameter of the blood collecting tube 132 at the position of attachment of the member main body 133 fitted with the elliptical O-ring 172.

Since the blood separating members according to the eighth and ninth embodiments have O-rings which are composed of elastic rubber, they have drawbacks in that their elasticity does not return to 100% after long-term changes, so that they can be permanently deformed by being pulled. Therefore, if used after the passage of a period of time such as one month after attaching the O-ring 134 to the member main body 133, the O-ring 134 may, after coming loose from the first groove 138, move above the blood separating member, i.e. above the thin leaf 137, without moving to the second groove 139 or the gap between the member main body 133 and the thin leaf 137.

This blood separating member 171 improves upon the drawbacks of the blood separating members of the eighth and ninth embodiments.

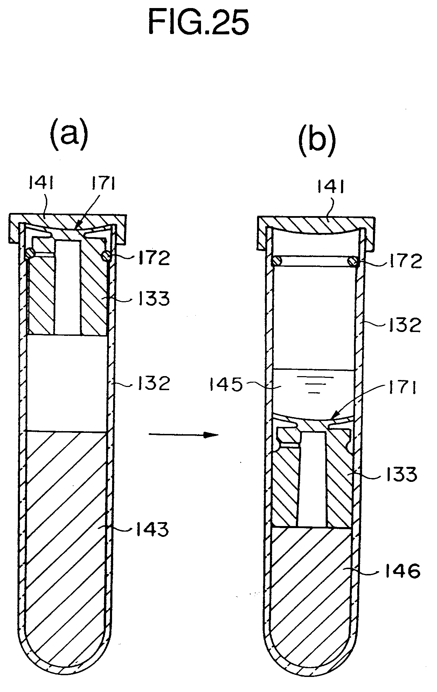

As shown in Fig. 25A, this blood separating member 171 can easily be attached in the blood collecting tube 132 after the elliptical O-ring 172 is fitted onto the member main body 133. In this case, when centrifugal separation is conducted after blood 143 is injected into the blood collecting tube 132 and the blood is coagulated, the member main body 133 is released from the elliptical O-ring 172 and drops, but the elliptical O-ring 172 remains stopped at a designated position on the blood collecting tube 132 as shown in Fig. 25B. Once the member main body 133 is released, the binding force from the inside stops working on this elliptical O-ring 172, which then presses against the inner wall of the blood collecting tube 132 by attempting to return to its original elliptical shape, as a result of which the elliptical O-ring 172 is affixed to the inner wall of the blood collecting tube 132.

In this case, if an O-ring formed from a plastic such as polyethylene or polypropylene and having an inner circumference slightly smaller than the inner wall of the blood collecting tube 132 is used instead of the elliptical O-ring 172, the member main body 133 can be easily attached by pushing into the blood collecting tube 132 when the member main body 133 fitted with the O-ring is attached to the blood collecting tube 132; however, when the member main body 133 has been released from the O-ring and dropped after centrifugal separation is started, the O-ring is stopped at a designated position in the blood collecting tube 132 but is not affixed thereto. Therefore, the O-ring easily moves from the designated position with only slight vibrations on the blood collecting tube 132, so that it is always oscillating and there is a risk of causing considerable problems in subsequent operations.

In the above manner, by using an elliptical O-ring 172 composed of a plastic such as polyethylene or polypropylene, having the overall shape of an elliptical ring and a circular cross-section, it is possible to prevent mishaps when the O-ring is sinking with the member main body 133 during centrifugal separation, and to stop the elliptical O-ring 172 at the designated position in the blood collecting tube 132.

Hereinbelow, an experimental example performed to clarify the effects of the blood separating member 171 will be explained.

(Experimental Example)

A member main body 133 was formed from an elastomer having a specific gravity of 1.09 and a rubber hardness of 45 degrees (JIS K-6301). This member main body 133 had a weight of approximately 2.0 g, a height of 18 mm, a bottom portion outer diameter of 12.9 mm, a top portion outer diameter of 12.0 mm, and a space portion 135 which opened downward with a diameter of 4 ~ 4.6 mm. Additionally, the curved top surface 173 had a radius of curvature of 23.6 mm. Additionally, the thin leaf 137 had a thickness of 0.75 mm, and an outer diameter of 13.6 mm. Additionally, through holes 152 having diameters of 0.5 mm which pass through the member main body 133 in a horizontal direction were formed at upper positions above the space portion 135 of the member main body 133, so as to allow the air lower than the through holes 152 to escape from the space portion 135. Furthermore, a groove 138 for fitting an elliptical O-ring 172 was formed at the position of the through holes 152. The diameter at the bottom of the groove 138 was 11.2 mm, and the cross-sectional surface of this groove 138 was in the shape of a semicircle of 0.7R.

The elliptical O-ring 172 was formed from polypropylene. This elliptical O-ring was an elliptical ring having an inner ellipse with a minor diameter of 10.6 mm and a major diameter of 12.4 mm, and an outer ellipse with a minor diameter of 13.2 mm and a major diameter of 15.0 mm, and having a circular cross-section with a diameter of 1.3 mm.

Additionally, the blood collecting tube 132 was formed from polyethylene terephthalate material, having an effective capacity of 10 ml, a height of 100 mm, an upper portion outer diameter of 16.0 mm, an inner diameter at the same location of 13.4 mm, with the top end of the mouth portion being machined to enlarge the inner diameter to 14.2 mm down to a depth of 6 mm.

At this time, the circumference at the bottom of the groove 138 of the member main body 133 was $3.14 \times 11.2 = 35.2$ mm, the inner circumference of the elliptical O-ring 172 was approximately $3.14 \times (10.2 + 12.4)/2 = 36.1$ mm, the outer circumference was 44.3 mm, and the circumference of the blood collecting tube 132 at the location where the member main body 133 fitted with the elliptical O-ring 172 is attached was $3.14 \times 14.2 = 44.6$ mm.

After the elliptical O-ring 172 was fitted into this member main body 133, it was easy to attach inside the blood collecting tube 132. Next, upon applying a centrifugal force of 1000 g in this empty state using a centrifuge, it was confirmed by the noise that the member main body 133 dropped inside the blood collecting tube 132 at a value of 800 g. When the blood collecting tube 132 was withdrawn after centrifugation, the elliptical O-ring 172 was affixed at a designated position on the blood collecting tube 132. Additionally, although the blood collecting tube 132 was inverted and vibrated, the elliptical O-ring 172 was not moved by small disturbances, and remained firmly attached to the inner wall of the blood collecting tube 132.

While an elliptical O-ring with a circular cross-section was used in the above embodiment, the cross-sectional shape of the elliptical O-ring is not necessarily restricted to that of the above embodiment, and various cross-sectional shapes are capable of being employed. For example, the elliptical O-ring can have a rectangular cross-section, that is, it may be an elliptical rectangular O-ring.

As explained above, during the handling of liquid samples, the component separating member and the component separating tube provided therewith according to the present invention completely ensures an airtight seal above and below a member when the member is attached to a container, without the risk of the separating member adhering to the wall surface of a container such as a blood collecting tube even when letting stand for long periods of time; as a result, it is useful in allowing more precise analysis values to be determined, and is especially suited for use in centrifugal separation operations wherein serum and clot are completely separated.

## Claims

1. A component separating member characterized by comprising:

   a member main body inserted into a tubular container; and
   at least one ring-shaped elastic member, provided at an outer circumferential portion of an upper portion of said member main body, which presses against an inner wall of said container and is impermeable to liquids.

2. A component separating member characterized by comprising:

   at least one elastic portion, formed at an outer circumferential portion of an upper portion of a member main body inserted into a tubular container, which completes a circuit in the circumferential direction and presses against the inner wall of said container.

3. A component separating member according to claim 2, wherein said elastic portion comprises a thin leaf extending horizontally outward from said outer circumferential portion.

4. A component separating member according to claim 2, wherein said elastic portion extends upward and outward from said outer circumferential portion, and has an inner circumferential surface composed of a ring-shaped thin leaf which recedes from said outer circumferential portion in an upward direction.

5. A component separating member according to one of claims 1 through 4, wherein a space portion which opens in a downward direction is formed coaxially on said member main body.

6. A component separating member according to claim 5, wherein a plurality of slits which open in a downward direction are formed in said member main body.

7. A component separating member according to claim 6, wherein top ends of said slits are positioned below a top end of said space portion.

8. A component separating member according to either claim 2 or claim 3, wherein top surfaces of said elastic portion and said member main body are curved into concave surfaces.

9. A component separating member according to either claim 2 or claim 3, wherein a bottom surface of said elastic portion is curved into a concave surface.

**10.** A component separating member according to either claim 2 or claim 3, wherein top surfaces of said elastic portion and said member main body are inclined with respect to the axis of said member main body.

**11.** A component separating member according to one of claims 8, 9 and 10, wherein said elastic portion has plasticity.

**12.** A component separating tube characterized by comprising:

a tubular container main body;
a tubular member provided at a mouth portion of said container main body and having an inner diameter slightly less than an inner diameter of said mouth portion; and
a component separating member according to one of claims 1 through 11, which is inserted into said tubular member and presses against an inner wall of said tubular member.

**13.** A component separating tube according to claim 12, wherein a thin sheet is adhered to a top surface of said component separating member.

**14.** A component separating tube according to claim 12, wherein an inner wall of said container main body has a tapered tube diameter.

**15.** A component separating tube according to one of claims 12, 13 and 14, wherein a plurality of particles, which have a specific gravity lighter than the heavier component of a liquid sample to be separated and which are inert with respect to the components, are inserted into said container main body along with said component separating member.

**16.** A component separating tube according to claim 15, wherein a material, having blood coagulation promoting agent dispersed in a water-soluble binder which is inert with respect to blood, is deposited onto the surfaces of said particles.

**17.** A component separating tube according to claim 15, wherein the surfaces of said particles are treated with a treatment to promote a coagulation of blood.

**18.** A component separating member characterized by comprising:

a member main body inserted into a tubular container; and
a ring-shaped elastic member which is supported on an outer circumferential portion of said member main body so as to be capable of moving in an axial direction of said member main body, and which presses against an inner wall of said container;

wherein
a first groove for supporting said elastic member in a state of protrusion outward from said outer circumferential portion and a second groove at a position above said first groove for housing said elastic member are respectively formed on an outer circumferential portion of said member main body.

**19.** A component separating member according to claim 18, wherein an elastic portion which presses against an inner wall of said container is formed at an upper portion of said member main body.

**20.** A component separating member according to claim 18, wherein said elastic member is composed of an elliptical O-ring.

**21.** A component separating member according to one of claims 18, 19 and 20, wherein a through hole which connect said space portion with either said first groove or said second groove is formed in said member main body.

**22.** A component separating member according to claim 19, wherein top surfaces of said member main body and said elastic portion are curved into concave surfaces.

**23.** A component separating tube characterized by comprising:

a tubular container main body; and
a component separating member according to one of claims 18 through 22, which is stored inside said container main body and presses against an inner wall thereof.

**24.** A component separating tube according to claim 23, wherein the inner wall of said container main body has a tapered tube diameter.

# FIG.1

# FIG.2

(a)

(b)

# FIG.3

# FIG.4

# FIG.6

# FIG.5

# FIG.7

# FIG.8

# FIG.9

# FIG.10

## FIG.11

## FIG.12

## FIG.13

## FIG.14

# FIG.15

# FIG.16

# FIG.17

# FIG.18

# FIG.19

# FIG.20

(a)          (b)

## FIG.21

## FIG.22

# FIG.23

# FIG.24

# FIG.25

(a)

(b)

# FIG.26

# FIG.27

(a)  (b)

# FIG.28

# FIG.29

## INTERNATIONAL SEARCH REPORT

| International application No. |
| --- |
| PCT/JP96/00159 |

**A. CLASSIFICATION OF SUBJECT MATTER**

Int. Cl$^6$ G01N33/48

According to International Patent Classification (IPC) or to both national classification and IPC

**B. FIELDS SEARCHED**

Minimum documentation searched (classification system followed by classification symbols)

Int. Cl$^6$ G01N33/48

Documentation searched other than minimum documentation to the extent that such documents are included in the fields searched

Jitsuyo Shinan Koho 1926 - 1995
Kokai Jitsuyo Shinan Koho 1971 - 1995

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP, 49-47973, A (Becton Dickinson and Co.),<br>May 9, 1974 (09. 05. 74),<br>Full descriptions & BE, 797267, A<br>& DE, 2308485, A & GB, 1381020, A | 1-3, 9, 11<br>12-14 |
| X<br>Y | JP, 50-124261, A (Becton Dickinson and Co.),<br>September 30, 1975 (30. 09. 75)<br>Full descriptions & BE, 797267, A<br>& DE, 2308485, A & GB, 1381020, A | 1-3, 9, 11<br>12-17 |
| X<br>Y | JP, 55-126855, A (Terumo Corp.),<br>October 1, 1980 (01. 10. 80),<br>Full descriptions & EP, 17127, A<br>& US, 4294707, A | 1-3, 9, 11<br>12, 13,<br>15-17 |
| X<br>Y | JP, 49-103696, A (F. Hoffmann-La Roche & Co.,<br>AG.),<br>October 1, 1974 (01. 10. 74),<br>Lines 13 to 18, upper right column, page 4<br>& NL 7316347, A & DE, 2358493, A<br>& FR, 2213207, A | 1-5, 8-11<br>12, 13,<br>15 - 17 |

[X] Further documents are listed in the continuation of Box C.    [ ] See patent family annex.

| | |
| --- | --- |
| *  Special categories of cited documents:<br>"A"  document defining the general state of the art which is not considered to be of particular relevance<br>"E"  earlier document but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| February 20, 1996 (20. 02. 96) | March 12, 1996 (12. 03. 96) |

| Name and mailing address of the ISA/ | Authorized officer |
| --- | --- |
| Japanese Patent Office | |
| Facsimile No. | Telephone No. |

Form PCT/ISA/210 (second sheet) (July 1992)

**INTERNATIONAL SEARCH REPORT**

| International application No. |
|---|
| PCT/JP96/00159 |

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP, 56-130656, A (Terumo Corp.),<br>October 13, 1981 (13. 10. 81),<br>Full descriptions (Family: none) | 1-5, 9, 11<br>12, 13,<br>15 - 17 |
| X<br><br>Y | JP, 50-157573, U (Matsushita Electric Ind. Co.,<br>Ltd.),<br>December 26, 1975 (52. 11. 75),<br>Full descriptions, drawings (Family: none) | 1-3, 6, 7,<br>9, 11<br>12, 13,<br>15 - 17 |
| Y | JP, 63-315161, A (Andronic Devices, Ltd.),<br>December 22, 1988 (22. 12. 88),<br>Fig. 3 & EP, 285076, A & AU, 8814041, A<br>& US, 4828716, A | 12, 13, 14,<br>15, 16, 17 |
| Y | JP, 53-9594, A (Andrew F.V.),<br>January 28, 1978 (28. 01. 78),<br>Lines 15 to 19, lower right column, page 2,<br>Fig. 4 (Family: none) | 13 |
| Y | JP, 57-135962, U (Ono Pharmaceutical Co., Ltd.),<br>August 25, 1982 (25. 08. 82),<br>Claim (Family: none) | 15, 16, 17 |
| Y | JP, 57-1326, A (Terumo Corp.),<br>January 6, 1982 (06. 01. 82),<br>Claim (Family: none) | 15, 16, 17 |
| Y | JP, 5-99917, A (Terumo Corp.),<br>Arpil 23, 1993 (23. 04. 93),<br>Claim (Family: none) | 15, 16, 17 |

Form PCT/ISA/210 (continuation of second sheet) (July 1992)